# DEMANDE DE BREVET EUROPEEN

(11) **EP 1 671 676 A1**
(43) Date de publication de la demande: **21.06.2006**
(21) Numéro de dépôt: 05292561.7
(22) Date de dépôt: 02.11.2005
(51) Int. Cl.: A61Q 17/04, A61K 8/04, A61K 8/42, A61K 8/35, A61K 8/46, A61K 8/49

(54) **Composition solaire comprenant au moins un filtre UV hydrophile et au moins une hydroxyalkyluree**

(30) Priorité: 20.12.2004 FR 0453076
(71) Demandeur: L'ORÉAL, 75008 Paris (FR)
(72) Inventeur: Candau, Didier, 91570 Bièvres (FR)
(74) Mandataire: Miszputen, Laurent

(57) **Abrégé**

L'invention concerne une composition comprenant dans un support cosmétiquement acceptable du type émulsion comprenant au moins un système filtrant les radiations UV, caractérisée par le fait qu'elle contient :
(a) au moins un filtre UV hydrophile et
(b) au moins une hydroxyalkylurée de formule (I) suivante. 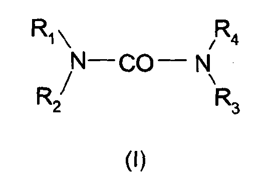
dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que l'un de ses sels, solvats ou isomères.

L'invention concerne également l'utilisation d'une hydroxyalkylurée de formule (I) que l'on détaillera ci-après dans une composition sous forme d'émulsion comprenant au moins un filtre UV hydrophile, pour augmenter l'efficacité de protection solaire.

L'invention concerne également l'utilisation d'une hydroxyalkylurée de formule (I) que l'on détaillera ci-après dans une composition sous forme d'émulsion comprenant au moins un filtre UV hydrophile, pour augmenter améliorer la répartition du filtre sur la matière kératinique.

## Description

L'invention concerne une composition comprenant dans un support cosmétiquement acceptable du type émulsion comprenant au moins un système filtrant les radiations UV, caractérisée par le fait qu'elle contient :
(a) au moins un filtre UV hydrophile et
(b) au moins une hydroxyalkylurée particulière.

L'invention concerne également l'utilisation d'une hydroxyalkylurée de formule particulière dans une composition sous forme d'émulsion comprenant au moins un filtre UV hydrosoluble, pour augmenter le facteur de protection solaire.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

Les rayons UVA et UVB doivent donc être filtrés et il existe actuellement des compositions cosmétiques protectrices de l'épiderme humain renfermant des filtres UVA et UVB.

Ces compositions antisolaires se présentent assez souvent sous la forme d'une émulsion, de type huile-dans-eau (c'est à dire un support cosmétiquement et/ou dermatologiquement acceptable constitué d'une phase continue dispersante aqueuse et d'une phase discontinue dispersée grasse) ou eau-dans-huile (phase aqueuse dispersée dans une phase grasse continue), qui contient, à des concentrations diverses, un ou plusieurs filtres organiques classiques lipophiles et/ou des filtres organiques classiques hydrophiles capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV. Dans de telles émulsions, les filtres hydrophiles sont présents dans la phase aqueuse et les filtres lipophiles sont présents dans la phase grasse.

Cependant ce type de formulation antisolaire limite le choix des filtres, notamment lorsqu'il s'agit des filtres hydrophiles car les compositions contenant ces filtres ont tendance, après l'application à présenter un étalement hétérogène et une mauvaise répartition des filtres à la surface de la peau qui aboutit à une efficacité de protection insuffisante.

On a déjà proposé d'incorporer des nanopigments d'oxyde titane dans une lotion

Or, à la suite d'importantes recherches menées dans le domaine de la photoprotection évoqué ci-dessus, la Demanderesse a maintenant découvert, de façon inattendue et surprenante, qu'en ajoutant, dans un support émulsion contenant au moins un filtre UV hydrophile, une hydroxyalkylurée de formule (I) que l'on détaillera ci-après, il était possible d'obtenir des compositions antisolaires dont l'application était sensiblement améliorée et qui présentait une efficacité améliorée. Les compositions antisolaires contenant une telle association présentent en outre une bonne rémanence à l'eau, à la transpiration et aux lavages ainsi qu'une bonne persistance dans le temps.

Dans la suite de la présente description, on entend par « système filtrant les radiations UV » par un agent filtrant les radiations UV constitué soit d'un composé organique ou minéral unique filtrant les radiations UV soit un mélange de plusieurs composés organiques ou minéraux filtrant les radiations UV, par exemple mélange comprenant un filtre UVA et un filtre UVB.

Dans la suite de la présente description, on entend par « filtre UV hydrophile» par tout agent filtrant les radiations UV susceptible d'être complètement dissous à l'état moléculaire dans la phase aqueuse de l'émulsion ou bien d'être solubilisé sous forme colloïdale (par exemple sous forme micellaire) dans la phase aqueuse de l'émulsion.

Cette découverte est à la base de la présente invention.

Ainsi, conformément à l'un des objets de la présente invention, il est maintenant proposé une composition cosmétique ou dermatologique comprenant dans un support cosmétiquement acceptable du type émulsion au moins un système filtrant les radiations UV, caractérisée par le fait qu'elle contient :
(a) au moins un filtre UV hydrophile et
(b) au moins une hydroxyalkylurée de formule (I) que l'on détaillera ci-après.

L'invention concerne également l'utilisation d'une hydroxyalylrée de formule (I) que l'on détaillera ci-après dans une composition sous forme d'émulsion comprenant au moins un filtre UV hydrophile, pour augmenter l'efficacité de protection solaire.

L'invention concerne également l'utilisation d'une hydroxyalylrée de formule (I) que l'on détaillera ci-après dans une composition sous forme d'émulsion comprenant au moins un filtre UV hydrophile, pour augmenter améliorer la répartition du filtre sur la matière kératinique (peau, cils, sourcils, muqueuses, ongles).

D'autres caractéristiques, aspects et avantages de l'invention apparaîtront à la lecture de la description détaillée qui va suivre.

Les hydroxyalkyl urées conforment à l'invention sont choisies par celles répondant à la formule générale (I) : 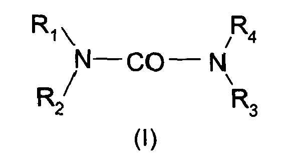 dans laquelle R_{1'} R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que leurs sels, solvats et isomères.

Dans la formule (I), parmi les groupes alkyle, on peut notamment citer les groupes méthyle, éthyle, n-propyle, isopropyle, n-butyle, iso-butyle et tert-butyle.

Les composés de formule (I) préférés sont ceux ne renfermant qu'un seul groupe hydroxyalkyle, c'est-à-dire ceux pour lesquels R₁ est un groupe hydroxyalkyle et R2, R3 et R4 représentent, indépendamment les uns des autres, un atome d'hydrogène ou un groupe alkyle en C₁-C₄. On préfère plus particulièrement les composés de formule (I) pour lesquels R₁ est un groupe hydroxyalkyle et R₂, R₃ et R₄ représentent chacun un atome d'hydrogène.

Parmi les groupes hydroxyalkyle, on préfère ceux contenant un seul groupe hydroxyle et en particulier les groupes hydroxyéthyle, hydroxypropyle, hydroxybutyle, hydroxypentyle et hydroxyhexyle. Le groupe hydroxyéthyle est préféré.
Comme composés de formule (I) préférés, on peut citer la N-(2-hydroxyéthyl)-urée ; la N-(2-hydroxypropyl)-urée ; la N-(3-hydroxypropyl)-urée ; la N-(2,3-dihydroxypropyl)- urée ; la N-(2,3,4,5,6-pentahydroxyhexyl)- urée; la N-méthyl-N-(1,3,4,5,6-pentahydroxy-2-hexyl)- urée ; la N-méthyl-N'-(l-hydroxy-2-méthyl-2-propyl)- urée ; la N-(1-hydroxy-2-méthyl-2-propyl)- urée ; la N-(1,3-dihydroxy-2-propyl)- urée ; la N-(tris-hydroxyméthyl-méthyl)-urée ; la N-éthyl-N'-(2-hydroxyéthyl)- urée ; la N,N-bis-(2-hydroxyéthyl)- urée ; la N,N'-bis-(2-hydroxyéthyl)- urée ; la N,N-bis-(2-hydroxypropyl)- urée ; la N,N'-Bis-(2-hydroxypropyl)- urée ; la N,N-Bis-(2-hydroxyéthyl)-N'-propyl- urée; la N,N-Bis-(2-hydroxypropyl)-N'-(2-hydroxyéthyl)- urée ; la N-tert.Butyl-N'-(2-(hydroxyéthyl)-N'-(2-(hydroxypropyl)- urée; la N-(1,3-dihydroxy-2-propyl)-N'-(2-hydroxyéthyl)- urée; la N,N-Bis-(2-hydroxyéthyl)-N',N'-dimethyl- urée ; la N,N,N',N'-tetrakis-(2-hydroxyéthyl)- urée ; et la N',N'-Bis-(2-hydroxyéthyl)-N',N'-bis-(2-hydroxypropyl)- urée.

Un composé particulièrement préféré pour une utilisation dans la présente invention est la N-(2-hydroxyéthyl)-urée, ci-après désignée par "hydroxyéthyl urée".

Les hydroxyalkyl urées de formule (I) peuvent être préparées comme décrit dans la demande DE-27 03 185. Parmi celles-ci, l'hydroxyéthyl urée est en outre disponible dans le commerce, sous forme de mélange à 50% en poids dans l'eau, auprès de la société NATIONAL STARCH sous la dénomination commerciale Hydrovance®.

Parmi les sels, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. II peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (I).

Les hydroxyalkylurée conformes à l'invention sont de préférence présentes dans les compositions conformes à l'invention à des teneurs de 0,01 à 50% en poids et plus préférentiellement de 0,1 à 20 % et encore plus préférentiellement de 0,1 à 10% en poids par rapport au poids total de la composition.

Les filtres UV hydrophiles conformes à l'invention contiennent en général au moins un radical carboxylique ou sulfonique sous forme libre ou bien sous une forme neutralisée, partiellement ou totalement. De préférence, on choisira ceux présentant au moins un radical sulfonique.

Parmi les filtres UV hydrophiles utilisables selon l'invention, on peut citer ceux désignés ci-dessus sous leur nom INCI :
(1) les dérivés de p-aminobenzoïque (PABA) comme
   - PABA
   - Glyceryl PABA
   - PEG-25 PABA vendu sous le nom « UVINUL P25 » par BASF,
(2) les dérivés de benzophénone comportant au moins un radical sulfonique comme
   - Benzophenone-4 vendu sous le nom commercial « UVINUL MS40 » par BASF,
   - Benzophenone-5
   - Benzophenone-12
(3) les dérivés de benzylidène camphre comportant au moins un radical sulfonique comme par exemple :
   - Benzylidene Camphor Sulfonic Acid fabriqué sous le nom « MEXORYL SL» par CHIMEX,
   - Camphor Benzalkonium Methosulfate fabriqué sous le nom « MEXORYL SO » par CHIMEX,
   - Terephthalylidene Dicamphor Sulfonic Acid fabriqué sous le nom « MEXORYL SX » par CHIMEX,
(4) les dérivés de benzimidazole comportant au moins un radical sulfonique comme par exemple :
   - Phenylbenzimidazole Sulfonic Acid vendu notamment sous le nom commercial « EUSOLEX 232 » par MERCK,
   - les dérivés bis-benzoazolyle tels que décrits dans les brevets EP669323 et
      US 2,463,264 et plus particulièrement le composé Disodium Phenyl Dibenzimidazole Tetra-sulfonate vendu sous le nom commercial commercial « NEO HELIOPAN AP » par Haarmann et REIMER,
(5) les dérivés de cinnamate hydrophiles comme par exemple le DEA Methoxycinnamate.
(6) leurs mélanges.

Parmi ces filtres, les plus préférentiels sont choisis parmi
Terephthalylidene Dicamphor Sulfonic Acid
Benzophenone-4
Phenylbenzimidazole Sulfonic Acid
Disodium Phenyl Dibenzimidazole Tetra-sulfonate ainsi que leurs mélanges.

Les filtres hydrophiles conformes à l'invention sont de préférence présents dans les compositions selon l'invention à une teneur allant de 0,1 % à 30 % en poids et de préférence de 0,5 à 15 % , en poids, par rapport au poids total de la composition.

Les compositions conformes à l'invention peuvent également comporter en plus un ou plusieurs filtres UV organiques ou inorganiques actifs dans l'UVA et/ou l'UVB, liposolubles ou bien insolubles dans les solvants cosmétiques couramment utilisés.

Les filtres organiques complémentaires liposolubles sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate notamment ceux cités dans le brevet US5624663 ; les dérivés de benzimidazole ; les imidazolines ; les dérivés de l'acide p-aminobenzoïque (PABA) ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649, les 4,4-diarylbutadiènes tels que ceux décrits dans les demandes de brevet DE19755649 , EP916335, EP1133980, EP1133981 et EP-A-1008586 et leurs mélanges.

Comme exemples de filtres organiques actifs dans l'UV-A et/ou l'UV-B, on peut citer ceux désignés ci-dessus sous leur nom INCI :

Dérivés de l'acide para-aminobenzoigue :
Ethyl PABA,
Ethyl Dihydroxypropyl PABA,
Ethylhexyl Diméthyl PABA vendu notamment sous le nom « ESCALOL 507 » par ISP,

Dérivés salicyliques :
Homosalate vendu sous le nom « Eusolex HMS » par Rona/EM Industries,
Ethylhexyl Salicylate vendu sous le nom « NEO HELIOPAN OS » par Haarmann et REIMER,
TEA Salicylate, vendu sous le nom « NEO HELIOPAN TS » par Haarmann et REIMER,

Dérivés du dibenzoylméthane :
Butyl Methoxydibenzoylmethane vendu notamment sous le nom commercial « PARSOL 1789 » par HOFFMANN LAROCHE,
Isopropyl Dibenzoylmethane,

Dérivés cinnamiques :
Ethylhexyl Methoxycinnamate vendu notamment sous le nom commercial « PARSOL MCX » par HOFFMANN LAROCHE,
lsopropyl Methoxy cinnamate,
lsoamyl Methoxy cinnamate vendu sous le nom commercial « NEO HELIOPAN E 1000 » par HAARMANN et REIMER,
Cinoxate,
Diisopropyl Methylcinnamate,

Dérivés de β,β-diphénylacrylate :
Octocrylene vendu notamment sous le nom commercial « UVINUL N539 » par BASF,
Etocrylene, vendu notamment sous le nom commercial « UVINUL N35 » par BASF,

Dérivés de la benzophénone :
Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
le 2-(4-diéthylamino-2-hydroxybenzoyl)-benzoate de n-hexyle

Dérivés du benzylidène camphre :
3-Benzylidene camphor fabriqué sous le nom « MEXORYL SD» par CHIMEX,
4-Methylbenzylidene camphor vendu sous le nom « EUSOLEX 6300 » par MERCK ,

Dérivés de triazine:
Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V
2,4,6-tris(4'-amino benzalmalonate de dinéopentyle)-s-triazine
la 2,4,6-tris-(4'-amino benzalmalonate de diisobutyle)-s- triazine.

Dérivés de benzotriazole :
Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

Dérivés anthraniliaues :
Menthyl anthranilate vendu sous le nom commercial commercial « NEO HELIOPAN MA » par Haarmann et REIMER,

Dérivés d'imidazolines :
Ethylhexyl Dimethoxybenzylidene Dioxoimidazoline Propionate,

Dérivés de benzalmalonate :
Di-néopentyl 4'-méthoxybenzalmalonate
Polyorganosiloxane à fonctions benzalmalonate tel que le polysilicone-15 vendu sous la dénomination commerciale « PARSOL SLX » par HOFFMANN LAROCHE

4,4-diarvlbutadiène :
1, 1-dicarboxy-(2'2'-dimethyl-propyl)-4,4-diphenylbutadiene

Dérivés de benzoxazole :
2,4-bis-[5-1 (diméthylpropyl)benzoxazol-2-yl-(4-phenyl)-imino]-6-(2-ethylhexyl)-imino-1,3, 5-triazine vendu sous le nom Uvasorb K2A par Sigma 3V ;
et leurs mélanges.

Les filtres organiques complémentaires plus particulièrement préférés sont choisis parmi les composés suivants :
Ethylhexyl Salicylate,
Ethylhexyl Methoxycinnamate
Octocrylene,
Butyl Methoxydibenzoylmethane
Benzophenone-3,
le 2-(4-diéthylamino-2-hydroxybenzoyl)-benzoate de n-hexyle 4-Methylbenzylidene camphor,
2,4,6-tris(4'-amino benzalmalonate de dinéopentyle)-s-triazine
la 2,4,6-tris-(4'-amino benzalmalonate de diisobutyle)-s-triazine
Bis-Ethylhexyloxyphenol Methoxyphenyl Triazine
Ethylhexyl triazone,
Diethylhexyl Butamido Triazone,
Drometrizole Trisiloxane
Polysilicone 15
Di-néopentyl 4'-méthoxybenzalmalonate
1,1-dicarboxy-(2'2'-dimethyl-propyl)-4,4-diphenylbutadiene
2,4-bis-[5-1 (diméthylpropyl)benzoxazol-2-yl-(4-phenyl)-imino]-6-(2-ethylhexyl)-imino-1,3,5-triazine et leurs mélanges.

Parmi les filtres UV organiques insolubles complémentaires, on peut citer ceux décrits dans les demandes US 5,237,071, US 5,166,355, GB2303549, DE 197 26 184 et EP893119 et en particulier les dérivés de méthylène bis-(hydroxyphényl benzotriazole) comme le Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

Les agents photoprotecteurs inorganiques complémentaires sont choisis parmi les pigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium et leurs mélanges. Des agents d'enrobage classiques sont par ailleurs l'alumine et/ou le stéarate d'aluminium. De tels pigments d'oxydes métalliques, enrobés ou non enrobés, sont en particulier décrits dans les demandes de brevets EP 518 772 et EP 518 773.

Les agents photoprotecteurs additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Les filtres complémentaires sont de préférence présents dans les compositions selon l'invention à une teneur allant de 0,1 % à 30 % en poids et de préférence de 0,5 à 15 % , en poids, par rapport au poids total de la composition.

Les compositions selon l'invention sont sous forme d'émulsions obtenues par dispersion d'une phase grasse (appelée aussi phase huileuse) dans une phase aqueuse (H/E) ou inversement (E/H) ou d'émulsions multiples (par exemple E/H/E ou H/E/H ou H/H/E). Elles peuvent être plus ou moins fluides et avoir l'aspect d'une crème blanche ou colorée, d'une pommade, d'un lait, d'une pâte, de bâtonnet solide et éventuellement être conditionnée en aérosol et se présenter sous forme de mousse ou de spray. Ces compositions sont préparées selon les méthodes usuelles.

La proportion de la phase huileuse de l'émulsion peut aller de 1 à 80 % en poids, de préférence de 2 à 50 % en poids et mieux de 2 à 40 % en poids par rapport au poids total de la composition. Les corps gras de la phase huileuse, notamment les huiles, et les émulsionnants et co-émulsionnants éventuellement présents, utilisés dans la composition sous forme d'émulsion sont choisis parmi ceux classiquement utilisés dans le domaine cosmétique ou dermatologique. L'émulsionnant et le co-émulsionnant ,quand ils sont présents, le sont généralement, en une proportion allant de 0,1 à 30 % en poids, de préférence de 0,3 à 20 % en poids et mieux de 0,5 à 15 % en poids par rapport au poids total de la composition. L'émulsion peut en outre, contenir des vésicules lipidiques en plus ou à la place des émulsionnants et/ou co-émulsionnants.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H, on peut citer par exemple les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90^{R} par la société Goldschmidt et le mélange de Polyglyceryl-4 isostearate/Cetyl dimethicone copolyol/Hexyl laurate vendu sous la dénomination Abil WE 09^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; les esters d'acides gras et de sorbitan oxyalkylénés comprenant par exemple de 20 à 100 OE, et par exemple ceux commercialisés sous les dénominations commerciales Tween 20 ou Tween 60 par la société Uniqema ; les éthers d'alcools gras oxyalkylénés (oxyéthylénés et/ou oxypropylénés) ; les esters de sucres, alcoxylés ou non, comme le stéarate de sucrose et comme le PEG-20 méthylglucose sesquistéarate ; les esters de sorbitan tels que le palmitate de sorbitan commercialisé sous la dénomination Span 40 par la société Uniqema ; les esters de diacide et d'alcool gras, tels que le tartrate de dimyristyle ; les mélanges de ces émulsionnants comme par exemple le mélange de stéarate de glycéryle et de stéarate de PEG-100 (nom CTFA : Glyceryl Stearate / PEG-100 Stearate) commercialisé sous la dénomination Arlacel 165 par la société Uniqema et sous la dénomination SIMULSOL 165 par la société SEPPIC ; ou le mélange de tartrate de dimyristyle, d'alcool cétéarylique, de Pareth-7 et de PEG-25 laureth-25, commercialisé sous la dénomination Cosmacol PSE par la société Sasol (nom CTFA : Dimyristyl tartrate / cetearyl alcool /12-15 Pareth 7 / PPG 25 laureth 25).

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique, ou les acides gras.

On peut aussi préparer des émulsions sans tensioactifs émulsionnants ou en contenant moins de 0,5 % du poids total de la composition, en utilisant des composés appropriés, pour stabiliser lesdites émulsions par exemple des polymères amphiphiles, des charges, des épaississants ou des gélifiants

Comme huiles utilisables dans la composition de l'invention, on peut utiliser par exemple les huiles hydrocarbonées d'origine animale, telles que le perhydrosqualène (ou squalane) ; les huiles hydrocarbonées d'origine végétale, telles que les triglycérides des acides caprylique/caprique comme ceux vendus par la société Stearineries Dubois ou ceux vendus sous les dénominations Miglyol 810, 812 et 818 par la société Dynamit Nobel ou encore les huiles d'origine végétale, par exemple les huiles de tournesol, de maïs, de soja, de courge, de pépins de raisin, de sésame, de noisette, d'abricot, de macadamia, d'arara, de coriandre, de ricin, d'avocat, l'huile de jojoba, l'huile de beurre de karité ; les huiles de synthèse ; les huiles de silicone comme les polyméthylsiloxanes (PDMS) volatiles ou non à chaîne siliconée linéaire ou cyclique, liquides ou pâteux à température ambiante ; les huiles fluorées telles que celles partiellement hydrocarbonées et/ou siliconées comme celles décrites dans le document JP-A-2-295912 ; les éthers tels que l'éther dicaprylique (nom CTFA : Dicaprylyl ether) ; et les benzoates d'alcools gras en C₁₂-C₁₅ (Finsolv TN de FINETEX) ; les dérivés arylalkyl benzoates comme le 2-phenylethyl benzoate (X-Tend 226 de ISP) ; les huiles amidées comme le N-lauroylsarcosinate d'isopropyle (ELDEW SL-205 de Ajimoto) et leurs mélanges.

La phase huileuse peut aussi comporter un ou plusieurs corps gras choisi par exemple parmi les alcools gras (alcool cétylique, l'alcool stéarylique, alcool cétéarylique), les acides gras (acide stéarique) ou les cires (paraffine, cires de polyéthylène, carnauba, cire d'abeilles).

La composition de l'invention peut également contenir un ou plusieurs solvants organiques qui peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des alcools monohydriques linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer les dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de düsopropyle, l'adipate de dioctyle, les benzoates d'alkyle.

Les compositions conformes à la présente invention peuvent également comprendre des adjuvants cosmétiques classiques choisis parmi les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, les charges, les polymères, les propulseurs, les agents alcalinisants ou acidifiants ou tout autre ingrédient habituellement utilisé dans le domaine cosmétique et/ou dermatologique.

Comme épaississants hydrophiles, on peut citer les polymères carboxyvinyliques tels que les carbopols (carbomers) et les Pemulen (Copolymère acrylate/C10-C30-alkylacrylate) ; les dérivés cellulosiques tels que l'hydroxyéthylcellulose ; les polysaccharides et notamment les gommes telles que la gomme de xanthane ; et leurs mélanges.

Comme épaississants lipophiles, on peut citer les argiles modifiées telles que le l'hectorite et ses dérivés, comme les produits commercialisés sous les noms de Bentone.

Comme conservateurs, on peut citer les esters de l'acide parahydroxybenzoïque encore appelés Parabens® (en particulier le méthyl parabène, l'éthyl parabène, le propyl parabène), le phénoxyéthanol, les libérateurs de formol comme par exemple l'imidazolidinyl urée ou la diazolidinyl urée, le digluconate de chlorhexidine, le benzoate de sodium, le caprylyl glycol, l'iodo propynyl butyl carbamate, le pentylène glycol, le bromure d'alkyl triméthylammonium tel que le bromure de myristyl-triméthylammonium (nom CTFA : bromure de Myrtrimonium), le bromure de dodécyl-triméthylammonium, le bromure d'hexadécyl-triméthylammonium, et leurs mélanges tel que le mélange vendu sous la dénomination Cetrimide® par la société FEF CHEMICALS.Le conservateur peut être présent dans la composition selon l'invention en une teneur allant de 0,001 à 10 % en poids, par rapport au poids total de la composition, notamment allant de 0,1 à 5 % en poids, et en particulier allant de 0,2 à 3 % en poids.

Comme charges qui peuvent être utilisées dans la composition de l'invention, on peut citer par exemple, les pigments ; la poudre de silice ; le talc ; les particules de polyamide et notamment celles vendues sous la dénomination ORGASOL par la société Atochem ; les poudres de polyéthylène ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres de polymethylmethacrylate telles que celles coomercialisées sous la dénomination MICROPEARL M 100 par la société Matsumoto ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; les poudres de polyuréthanne telles que la poudre de copolymère hexaméthylène diisocyanate/trimethylol hexyllactone commercialisée sous la dénomination Plastic Powder D-400 par la société Toshiba Pigment (Nom CTFA : HDI / Trimethylol Hexyllactone Crosspolymer) ; et leurs mélanges. Quand elles sont présentes, ces charges peuvent être en des quantités allant de 0,001 à 20 % en poids, de préférence de 0,1 à 10 % en poids et mieux de 1 à 5 % en poids par rapport au poids total de la composition.

Bien entendu, l'homme de l'art veillera à choisir le ou les éventuels composés complémentaires cités ci-dessus et/ou leurs quantités de manière telle que les propriétés avantageuses attachées intrinsèquement à l'association conforme à l'invention ne soient pas, ou substantiellement pas, altérées par la ou les adjonctions envisagées.

La composition selon l'invention est généralement adaptée à une application topique sur la peau et comprend donc généralement un milieu physiologiquement acceptable, c'est-à-dire compatible avec la peau et/ou ses phanères. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

La composition selon l'invention peut constituer un produit pour le soin de la peau, notamment pour le visage, le cou, le contour de l'oeil, le corps ; ou bien encore un produit de maquillage de la peau tel qu'un produit du teint (notamment fond de teint), un fard à paupières, un fard à joue, un eye-liner, un produit anticernes, un produit de maquillage du corps, un produit de protection solaire ou bien un produit de nettoyage de la peau. De manière préférentielle La composition selon l'invention sera un produit de protection solaire.

La composition est généralement non rincée, mais elle peut être rincée si elle constitue un produit de nettoyage notamment moussant.

L'invention a aussi pour objet un procédé de traitement cosmétique d'une matière kératinique telle que la peau, des cils, des sourcils, des ongles ou des muqueuses, caractérisé en ce qu'on applique sur la matière kératinique, une composition telle que définie ci-dessus.

Selon un autre aspect, l'invention concerne également un ensemble cosmétique comprenant :
i) un récipient délimitant au moins un compartiment, ledit récipient étant fermé par un élément de fermeture ; et
ii) une composition telle que décrite précédemment et disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Les compositions selon l'invention peuvent se présenter sous forme de lotions fluides vaporisables conformes à l'invention sont appliquées sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

L'invention va maintenant être décrite en référence aux exemples suivants donnés à titre illustratif et non limitatif. Dans ces exemples, sauf indication contraire, les quantités sont exprimées en pourcentages pondéraux.

On a réalisé les formulations solaires suivantes ; les quantités sont indiquées en pourcentages en poids :

| **Compositions** | **Exemple 1** | **Exemple 2** | **Exemple 3** |
|---|---|---|---|
| **PHASE A** | | | |
| Polydiméthylsiloxane | 0,5 | 0,5 | 0,5 |
| Conservateurs | 1,0 | 1,0 | 1,0 |
| Acide stéarique | 1,5 | 1,5 | 1,5 |
| Isohexadécane | 1,0 | 1,0 | 1,0 |
| Mélange monostéarate de glycéryle/stéarate-PEG (100 OE) | 1,0 | 1,0 | 1,0 |
| Benzoate d'alkyle en C₁₂/C₁₅ | 5,0 | - | 5,0 |
| Mélange cetylstéarylglucoside/alcool cetylstéarylique | 2,0 | 2,0 | 2,0 |
| Alcool cétylique | 0,5 | 0,5 | 0,5 |

| **PHASE B** | | | |
|---|---|---|---|
| Terephthalylidene Dicamphor Sulfonic Acid | 3 | - | - |
| Phenylbenzimidazole Sulfonic Acid | - | 3 | - |
| Benzophenone-4 | - | - | 4 |
| N-(2-hydroxyléthyl)urée | 5 | 10 | 5 |
| Sequestrant | 0,1 | 0,1 | 0,1 |
| Glycérine | 5,0 | 5,0 | 5,0 |
| Gomme de Xanthane | 0,2 | 0,2 | 0,2 |
| Phosphate de monocétyle | 1,0 | 1,0 | 1,0 |
| Eau désionisée | qsp 100 | qsp 100 | qsp 100 |

| **PHASE C** | | | |
|---|---|---|---|
| Copolymère acide acrylique/méthacrylate de stéaryle | 0,2 | 0,2 | 0,2 |
| Triéthanolamine | 0,65 | 0,65 | 0,65 |

### Mode opératoire

On chauffe la phase aqueuse (Phase B) contenant l'ensemble de ses ingrédients à 80°C au bain marie. On chauffe la phase grasse (Phase A) contenant l'ensemble de ses ingrédients à 80°C au bain marie. On émulsionne A dans B sous agitation de type rotorstator (appareil de la société Moritz). On incorpore la phase C et on laisse revenir à température ambiante sous agitation modérée. On introduit la triéthanolamine de façon à ajuster le pH à la valeur désirée en fin de fabrication.

## Revendications

**1.** Composition comprenant dans un support cosmétiquement acceptable du type émulsion comprenant au moins un système filtrant les radiations UV, **caractérisée par le fait qu'**elle contient :
(a) au moins un filtre UV hydrophile et
(b) au moins une hydroxyalkylurée de formule (I) 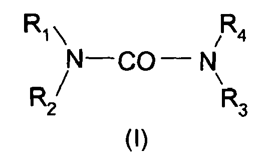 dans laquelle R₁, R₂, R₃ et R₄ représentent chacun indépendamment un atome d'hydrogène, un groupe alkyle en C₁-C₄ ou un groupe hydroxyalkyle en C₂-C₆ pouvant contenir de 1 à 5 groupes hydroxyle, au moins l'un des radicaux R₁-R₄ représentant un groupe hydroxyalkyle, ainsi que l'un de ses sels, solvats ou isomères.

**2.** Composition selon la revendication 1, **caractérisée en ce que** R₁ est un groupe hydroxyalkyle et R₂, R₃ et R₄ représentent, indépendamment les uns des autres, un atome d'hydrogène ou un groupe alkyle en C₁-C₄.

**3.** Composition selon la revendication 2, **caractérisée en ce que** R₁ est un groupe hydroxyalkyle et R₂, R₃ et R₄ représentent chacun un atome d'hydrogène.

**4.** Composition selon l'une quelconque des revendications 1 à 3, **caractérisée en ce que** le composé de formule (I) est choisi parmi la N-(2-hydroxyéthyl)-urée ; la N-(2-hydroxypropyl)-urée ; la N-(3-hydroxypropyl)-urée ; la N-(2,3-dihydroxypropyl)- urée ; la N-(2,3,4,5,6-pentahydroxyhexyl)- urée; la N-méthyl-N-(1,3,4,5,6-pentahydroxy-2-hexyl)-urée ; la N-méthyl-N'-(l-hydroxy-2-méthyl-2-propyl)- urée ; la N-(1-hydroxy-2-méthyl-2-propyl)- urée ; la N-(1,3-dihydroxy-2-propyl)- urée ; la N-(tris-hydroxyméthyl-méthyl)-urée ; la N-éthyl-N'-(2-hydroxyéthyl)- urée ; la N,N-bis-(2-hydroxyéthyl)- urée ; la N,N'-bis-(2-hydroxyéthyl)- urée ; la N,N-bis-(2-hydroxypropyl)- urée ; la N,N'-Bis-(2-hydroxypropyl)- urée ; la N,N-Bis-(2-hydroxyéthyl)-N'-propyl- urée; la N,N-Bis-(2-hydroxypropyl)-N'-(2-hydroxyéthyl)- urée ; la N-tert.Butyl-N'-(2-(hydroxyéthyl)-N'-(2-(hydroxypropyl)- urée ; la N-(1,3-dihydroxy-2-propyl)-N'-(2-hydroxyéthyl)- urée ; la N,N-Bis-(2-hydroxyéthyl)-N',N'-dimethyl- urée ; la N,N,N',N'-tetrakis-(2-hydroxyéthyl)- urée ; la N',N'-Bis-(2-hydroxyéthyl)-N',N'-bis-(2-hydroxypropyl)- urée.

**5.** Composition selon l'une quelconque des revendications 1 à 4, **caractérisée en ce que** l'hydroxyalkyl urée est la N-(2-hydroxyéthyl)-urée.

**6.** Composition selon l'une quelconque des revendications 1 à 5 où le ou les hydroxyalkylurées sont présentes à des teneurs allant de 0,01 à 50% en poids et plus préférentiellement de 0,1 à 20 % et encore plus préférentiellement de 0,1 à 10% en poids par rapport au poids total de la composition.

**7.** Composition selon l'une quelconque des revendications 1 à 6, où le filtre hydrophile contient au moins un radical carboxylique ou sulfonique sous forme libre ou bien sous une forme neutralisée, partiellement ou totalement.

**8.** Composition selon la revendication 7, où le filtre hydrophile contient au moins un radical sulfonique.

**9.** Composition selon l'une quelconque des revendications 1 à 6, où le filtre UV hydrophile est choisi parmi
(1) les dérivés de p-aminobenzoïque (PABA)
(2) les dérivés de benzophénone comportant au moins un radical sulfonique
(3) les dérivés de benzylidène camphre comportant au moins un radical sulfonique
(4) les dérivés de benzimidazole
(5) les dérivés de cinnamate hydrophiles
(6) leurs mélanges.

**10.** Composition selon la revendication 9, où le filtre UV hydrophile est choisi parmi
- PABA
- Glyceryl PABA
- PEG-25 PABA
- Benzophenone-4
- Benzophenone-5
- Benzophenone-12
- Benzylidene Camphor Sulfonic
- Camphor Benzalkonium Methosulfate
- Terephthalylidene Dicamphor Sulfonic Acid
- Phenylbenzimidazole Sulfonic Acid
- Disodium Phenyl Dibenzimidazole Tetra-sulfonate
- DEA Methoxycinnamate ou leurs mélanges.

**11.** Composition selon la revendication 10, où le filtre UV hydrophile est choisi parmi
- Terephthalylidene Dicamphor Sulfonic Acid
- Benzophenone-4
- Phenylbenzimidazole Sulfonic Acid
- Disodium Phenyl Dibenzimidazole Tetra-sulfonate ou leurs mélanges

**12.** Composition selon l'une quelconque des revendications 1 à 11, où le ou les filtres hydrophiles sont présents à des teneurs allant de 0,1 à 30% en poids et plus préférentiellement de 0,5 à 15% en poids par rapport au poids total de la composition.

**13.** Composition selon l'une quelconque des revendications 1 à 12, **caractérisée en ce qu'**elle constitue un produit pour le soin de la peau, un produit de maquillage de la peau, un produit de protection solaire, un produit de nettoyage de la peau.

**14.** Composition selon la revendication 13, **caractérisée en ce qu'**elle constitue un produit de protection solaire.

**15.** Procédé de traitement cosmétique d'une matière kératinique, **caractérisé par le fait qu'**on applique sur la matière kératinique, une composition cosmétique selon l'une quelconque des revendications 1 à 14.

**16.** Ensemble cosmétique comprenant
(i) un récipient délimitant au moins un compartiment, ledit récipient étant fermé par un élément de fermeture ; et
(ii) une composition selon l'une quelconque des revendications 1 à 14 et disposée à l'intérieur dudit compartiment.

**17.** Utilisation d'une hydroxyalkylurée selon l'une quelconque des revendications précédentes dans une composition sous forme d'émulsion comprenant au moins un filtre UV hydrophile, dans le but d'augmenter efficacité de protection solaire.

**18.** Utilisation d'une hydroxyalkylurée selon l'une quelconque des revendications précédentes dans une composition sous forme d'émulsion comprenant au moins un filtre UV hydrophile, dans le but d'améliorer la répartition du filtre sur la matière kératinique.
